(19) Europäisches Patentamt
European Patent Office
Office européen des brevets

(11) Veröffentlichungsnummer: **0 286 953 B1**

(12) # EUROPÄISCHE PATENTSCHRIFT

(45) Veröffentlichungstag der Patentschrift: **26.09.90**

(51) Int. Cl.[5]: **C07C 39/26**, C07C 43/23, C08J 3/02, C08G 63/60

(21) Anmeldenummer: **88105431.6**

(22) Anmeldetag: **06.04.88**

(54) **Verwendung von Fluormethylphenolen als Lösungsmittel für LC-Polymere, und neue Fluormethylphenole.**

(30) Priorität: **15.04.87 DE 3712817**

(43) Veröffentlichungstag der Anmeldung: **19.10.88 Patentblatt 88/42**

(45) Bekanntmachung des Hinweises auf die Patenterteilung: **26.09.90 Patentblatt 90/39**

(84) Benannte Vertragsstaaten: **BE CH DE FR GB IT LI NL**

(56) Entgegenhaltungen:
**EP-A- 0 000 542**
**US-A- 4 157 344**

**PATENT ABSTRACTS OF JAPAN, Band 11, Nr. 18 (C-398) (2465), 17. Januar 1987; & JP-A-61 194 042**

(73) Patentinhaber: **BAYER AG, D-5090 Leverkusen 1 Bayerwerk(DE)**

(72) Erfinder: **Kuhn, Rainer, Dr., Fasanenweg 13, D-5068 Odenthal(DE)**
Erfinder: **Marhold, Albrecht, Dr., Carl-Duisberg-Strasse 329, D-5090 Leverkusen 1(DE)**
Erfinder: **Dicke, Hans-Rudolf, Dr., Bodelschwinghstrasse 16, D-4150 Krefeld(DE)**

EP 0 286 953 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft die Verwendung bestimmter Fluormethylphenole als Lösungsmittel für sog. LC (liquid-crystalline)-Polymere. Die Erfindung betrifft weiterhin einige neue Fluormethylphenole, die sich für diesen Zweck eignen.

LC-Polymere, insbesondere LC-Polyester, erlangen aufgrund ihrer außergewöhnlichen Eigenschaften zunehmend Bedeutung auf dem Gebiet thermoplastischer Konstruktionswerkstoffe, hochfester Fasern und Filamente und hitzebeständiger Beschichtungen. In der Praxis auftretende Schwierigkeiten bei der Handhabung vieler LC-Polymerer kommen dadurch zustande, daß einerseits einige dieser hochmolekularen Verbindungen schon unter normalen Umständen in üblichen organischen Lösungsmitteln unlöslich sind und andererseits in üblichen organischen Lösungsmitteln noch lösliche Polymere durch eine Nachkondensation, die zwecks Erhöhung des Molekulargewichts und damit zur Verbesserung mechanischer Eigenschaften ausgeführt wird, schwerlöslich oder gar unlöslich werden.

Für schwerlösliche LC-Polymere wird als spezielles Lösungsmittel gelegentlich Pentafluorphenol empfohlen (R.W. Lenz, "Synthetic routes to liquid crystalline polymers" in "Recent Advances in Liquid Crystalline Polymers", Elsevier, New York 1985). Jedoch versagt auch dieses Lösungsmittel in vielen Fällen.

Überraschenderweise wurde nun gefunden, daß bestimmte Trifluormethylphenole dem Pentafluorphenol hinsichtlich der Lösefähigkeit von schwerlöslichen LC-Polymeren überlegen sind.

Gegenstand der Erfindung ist daher die Verwendung von Verbindungen der Formel

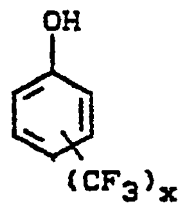

(I)

worin

x eine ganze Zahl von 1 bis 5, vorzugsweise von 2 bis 3, bedeutet,

wobei die Verbindungen I auch zusätzlich bis zu (5-x) Substituenten aus der Reihe

(1) $C_1$-$C_4$-Alkyl,

(2) Halogen aus der Reihe Fluor, Chlor,

(3) Mono- oder Polyfluor-$C_1$-$C_4$-alkyl (vorzugsweise Mono- oder Difluormethyl), das durch Wasserstoff-, Chlor- oder Bromatome substituiert sein kann,

(4) $C_1$-$C_4$-Alkoxy, das gegebenenfalls durch Fluor oder Chlor substituiert ist,

tragen können,

als Lösungsmittel für LC-Polymere.

Verbindungen I mit Substituenten aus der Kategorie (3) sind bevorzugt.

Bevorzugte Verbindungen I sind beispielsweise Phenole und Kresole mit 1 bis 3 $CF_3$-Substituenten, Phenole mit 1 bis 2 $CF_3$-Substituenten und 3 bis 4 Halogenatomen, Phenole mit 1 bis 2 $CF_3$-Substituenten und 1 bis 2 $C_1$-$C_4$-Alkoxygruppen, Phenole mit 1 bis 2 $CF_3$-Substituenten und 1 bis 2 $CHF_2$- oder $CF_2Cl$-Gruppen und Phenole mit 1 bis 2 $CF_3$-Substituenten und 1 bis 2 $OCF_3$-Gruppen.

Beispiele besonders bevorzugter Verbindungen I sind

(a) m-Trifluormethylphenol,
(b) p-Trifluormethylphenol,
(c) 2-Chlor-4-trifluormethylphenol,
(d) 2,6-Dichlor-4-trifluormethylphenol,
(e) 2-Isopropyl-5-trifluormethylphenol,
(f) 2,4,6-Tribrom-5-trifluormethylphenol,
(g) 2-Chlor-3,4,5-trifluor-6--trifluormethylphenol,
(h) 2-Chlor-3,5,6-trifluor-4-trifluormethylphenol,
(i) 2,3,5,6-Tetrafluor-4-trifluormethylphenol,
(k) 3-Trifluormethyl-4-trifluormethoxyphenol,
(l) 3-Trifluormethyl-4-difluorchlormethoxyphenol,
(m) 2,4,5-Tris-trifluormethylphenol und insbesondere
(n) 3,5-Bis-trifluormethylphenol.

Die Verbindungen I und Verfahren zu ihrer Herstellung sind größtenteils bekannt; vgl. beispielsweise FR 1 469 596, SU 520 343, DE 2 016 624, US 2 489 423, 2 547 679; J. Res. Nat. Bur. Std. 67 A (5), 481-493 (1963), Chem. Abstr. <u>60</u>, 9170 c (1964), Jzv. Sib. Obd. Akad. Nauk. SSR, Ges. Khim. Nauk (5), 95-102 (1982), Bull. Acad. Roy. Belg. 1913, 241; J. Org. Chem. 16, 586 (1951), J. Amer. Chem. Soc. 71, 4148 (1949), 69, 947 (1947), 73, 3470 (1951).

Im allgemeinen werden die entsprechenden Aniline diazotiert und zum Phenol verkocht, oder die entsprechenden Chlorbenzole werden mit Alkalilauge bei erhöhter Temperatur unter Chlorwasserstoffabspaltung zum gewünschten Phenol umgesetzt.

Einige der als Lösungsmittel geeigneten Verbindungen I sind aber auch neu. Weiterer Gegenstand der Erfindung sind daher Trifluormethylphenole der Formel

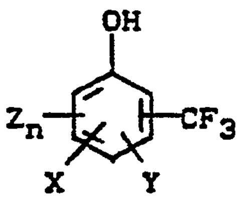

(II)

worin

X Fluor-$C_1$–$C_4$-alkyl oder Fluor-$C_1$–$C_4$-alkoxy,
Y Fluor, Chlor, Fluor-$C_1$–$C_4$-alkyl oder Fluor-$C_1$–$C_4$-alkoxy,
Z Fluor oder Chlor und
n Null, 1 oder 2
bedeuten mit Ausnahme der Kombination X = $CF_3$, Y = Fluor oder Chlor und n = Null.

Bevorzugte Verbindungen entsprechen der Formel (IIa)

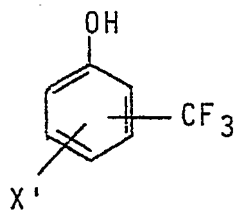

(IIa)

worin X' $OCF_2Cl$ bedeutet.

Bevorzugte Verbindungen der Formeln (II) und (IIa) sind 2,4,5-Tris-trifluormethylphenol und 3-Trifluormethyl-4-difluorchlormethoxyphenol.

Verbindungen ähnlicher Struktur sind in US-A 4 157 344 enthalten.

Die neuen Verbindungen können analog den Verfahren zur Herstellung der bekannten Verbindungen I hergestellt werden, also z.B. durch Diazotierung und anschließendes Verkochen der entsprechenden Aniline oder durch Umsetzung der entsprechenden Halobenzole mit Alkalilauge bei erhöhter Temperatur.

LC-Polymere sind hinreichend bekannt, vgl. z.B. F.E. Mc Farlane et al., Liquid Crystal Polymers II, Contemporary Topics in Polymer Science, Vol. 2, Plenum Publishing Corporation, 1977;

W.J. Jackson und H.F. Kuhfuss, J. Polymer Science, Polymer Chem. Ed. 14, 2042 (1976);

W.C. Wooten et al. in A. Ciferri "Ultra-high Modulus Polymers", Applied Science Publ., London 1979, S. 362 f.;

A. Blumenstein et al., "Liquid Crystalline Order in Polymers", Academic Press 1978;

J. Preston, Angew. Makromol, Chem. 109/110, S. 1-19 (1982);

A. Cifferri, W. R. Krigbaum, R. B. Meyer "Polymer Liquid Crystals", Academic Press, New York, 1982;

P. J. Flory, I. Uematsu, S. P. Papkov, CH. Ober und R. W. Lenz, Advances in Polymer Science 59 (1984);

B. Wunderlich, J. Grebowicz, M. G. Dobb. J. Mc Intyre, H. Finkelmann, G. Rehage, V. P. Shibaev und N. Plate, Advances in Polymer Science 60/61 (1984);

EP 1185, 1340, 8855, 11 640, 15 856, 17 310, 18 145, 18 709, 22 344, 44 205, 49 615;

US 3 991 013, 3 991 014, 4 066 620, 4 067 852, 4 083 829, 4 107 143;

US 3 991 013, 3 991 014, 4 066 620, 4 067 852, 4 083 829, 4107 143;

WO 79/797, 79/1034, 79/1040.

Der flüssig-kristalline Zustand von Polymerschmelzen läßt sich mit Hilfe eines Polarisationsmikroskops untersuchen: Für die Untersuchungen ist das Okular mit einem Aufsatz ausgerüstet, der eine Fotodiode im Brennpunkt der Okularlinse angeordnet enthält. Anhand eines nachgeschalteten Meßverstärkers mit Regeleinrichtung wird der Meßwert am eingeschalteten Mikroskop bei parallel angeordneten Nicolschen Prismen in Abwesenheit einer Materialprobe auf 100 Skalenteile eingestellt. Bei gekreuzten Nicolschen Prismen ergibt sich dann ein Wert von 0,01 Skalenteilen.

Die Schichtdicke der untersuchten Polymerschmelzen beträgt 100 µm.

Die Untersuchung der Polymeren erfolgt nach dem Aufschmelzen der Proben bei Temperaturen zwischen 200 und 400°C. Sofern in diesem gesamten Bereich oder in einem Teil davon eine Aufhellung der zwischen den gekreuzten Nicolschen Prismen beobachteten Schmelze auftritt, wird das Polymere als flüssigkristallin eingestuft.

Die flüssig-kristallinen Polymeren zeigen in der Meßanordnung Werte über 1 Skalenteil, meist Werte von 3 bis 90 Skalenteilen. Für amorphe Schmelzen, z.B. aromatische Polycarbonate, werden dagegen Werte von weniger als 0,1 Skalenteil gefunden.

Die oben beschriebene Methode des thermo-optischen Tests (TOT) ist für eine Schnellbestimmung im Laboratorium besonders geeignet und liefert in nahezu allen Fällen zweifelsfreie Ergebnisse. In Zweifelsfällen kann es dagegen sinnvoll sein, das Vorhandensein flüssig-kristalliner Komponenten mittels Röntgenweitwinkelstreuung in der Schmelze nachzuweisen, wie es z.B. bei G.W. Gray und P.A. Windsor, "Plastic Crystals, Physico-Chemical Properties and Methods of Investigation", insbesondere Chapter 3, John Wiley & Sons, New York, Sydney, Toronto 1974 beschrieben ist.

Geeignete LC-Polymere sind beispielsweise

- Polyester,
- Polythiolester,
- Polyesteramide,
- Polyesterimide,
- Polyazomethine,
- Polyestercarbonate.

Bevorzugte LC-Polymere sind vollaromatische Polyester auf Basis von

(a) aromatischen Dicarbonsäuren,
(b) Diphenolen und gegebenenfalls
(c) aromatischen Hydroxycarbonsäuren,

wobei das Molverhältnis von Resten einkondensierter aromatischer Dicarbonsäuren zu Resten einkondensierter Diphenole 0,95 bis 1,05 beträgt und die Reste einkondensierter aromatischer Hydroxycarbonsäuren 0 bis 100 Mol-%, vorzugsweise 30 bis 80 Mol-%, insbesondere 50 bis 70 Mol-%, bezogen auf einkondensierte Reste (a) und (c), ausmachen.

Aromatische Dicarbonsäuren (a) sind alle jene Dicarbonsäuren, deren Carboxylgruppen unmittelbar an einen aromatischen Ring geknüpft sind.

Bevorzugte aromatische Dicarbonsäuren (a) entsprechen der Formel

HOOC-A-COOH(III)

worin

A einen bivalenten aromatischen Rest mit 6 bis 24 C-Atomen, vorzugsweise mit 6 bis 16 C-Atomen, bedeutet.

Bevorzugte aromatische Reste A sind solche, bei denen sich die beiden Bindungen zu den Carboxylgruppen koaxial in entgegengesetzte Richtungen erstrecken, wie beispielsweise in 1,4-Phenylen, 1,4-Naphthylen oder 4,4'-Biphenylen, oder bei denen die in entgegengesetzte Richtungen zeigenden Bindungen parallel zueinander verschoben sind, wie beispielsweise in 1,5-Naphthylen, 2,6-Naphthylen oder 3,5'-Biphenylen.

Auch aromatische Reste A, deren beiden Bindungen zu den Carboxylgruppen sich nicht koaxial oder parallel verschoben in entgegengesetzte Richtungen erstrecken, sind geeignet, sofern die beiden Bindungen einen Winkel von 45° bis weniger als 180° einschließen und nicht an direkt benachbarten Kohlenstoffatomen sitzen, wie beispielsweise in 1,3-Phenylen, 1,3-, 1,6-, 1,7- oder 2,7-Naphthylen oder 3,4'-Biphenylen.

Bevorzugte arcmatische Dicarbonsäuren a) sind z.B. 1,4-Naphthalindicarbonsäure, 1,5-Naphthalindicarbonsäure, 2,6-Naphthalindicarbonsäure, 4,4'-Biphenyl-dicarbonsäure, 3,3'-Biphenyl-dicarbonsäure, 4,4'-Diphenoxyethandicarbonsäure, 4,4'-Diphenylether-dicarbonsäure, Methylterephthalsäure, Methoxyterephthalsäure, Chlorterephthalsäure, 4-Chlornaphthalin-2,7-dicarbonsäure, 1,3-Naphthalindicarbonsäure, 1,6-Naphthalindicarbonsäure, 1,7-Naphthalindicarbonsäure, 2,7-Naphthalindicarbonsäure, 3,4'-Biphenyl-dicarbonsäure, 3,4'-Diphenyletherdicarbonsäure, 4-Methylisophthalsäure, 5-Methylisophthalsäure, 4,4-Dichlordiphenylether-3,3'-dicarbonsäure, 4,4'-Benzophenondicarbonsäure und 3,4'-Benzophenondicarbonsäure. Besonders bevorzugte aromatische Dicarbonsäuren (a) sind Iso- und Terephthalsäure.

Bevorzute Diphenole (b) entsprechen der Formel
HO-D-OH(IV)
worin
D einen zweiwertigen ein- oder mehrkernigen aromatischen Rest mit 6 bis 30 C-Atomen bedeutet, wobei D derart gebaut ist, daß die beiden OH-Gruppen direkt an je ein Kohlenstoffatom eines aromatischen Systems gebunden sind und die beiden Bindungen zu den phenolischen Hydroxygruppen einen Winkel von 45° bis 180° bilden. Für die Konstitution des Restes D gilt das gleiche wie für den aromatischen Rest A oben beschrieben worden ist.

Besonders bevorzugte Diphenole (b) sind z.B. Hydrochinon, 4,4'-Dihydroxydiphenyl, 4,4'-Dihydroxydiphenylether, 4,4'-Dihydroxydiphenylethan, 4,4'-Dihydroxydiphenoxyethan, 3,5'-Dihydroxydiphenyl, 3,5'-Dihydroxydiphenylether, 1,5-Dihydroxynaphthalin, 2,6-Dihydroxynaphthalin, 1,4-Dihydroxynaphthalin, Chlorhydrochinon, Bromhydrochinon, Methylhydrochinon, Phenylhydrochinon, Ethylhydrochinon, 2,2'-Dimethyl-4,4'-dihydroxydiphenyl, 3,3',5,5'-Tetramethyl-4,4'-dihydroxydiphenyl, 3,5'-Dimethoxy-4,4'-dihydroxydiphenylether, 1,2-(2-Chlor-4-hydroxyphonoxy)-ethan, 4-Methoxy-2,6-dihydroxynaphthalin-Resorcin, 3,4'-Dihydroxydiphenyl, 3,4'-Dihydroxydiphenylether, 3,4'Dihydroxydiphenoxyethan, 1,3-Dihydroxynaphthalin, 1,6-Dihydroxynaphthalin, 1,7-Dihydroxynaphthalin, 2,7-Dihydroxynaphthalin, 4-Chlorresorcin, 4-Bromresorcin, 4-Methylresorcin, 4-Phenylresorcin, 4-Ethoxyresorcin, 2,5-Dichlor-1,6-dihydroxynaphthalin und 4-Methoxy-2,7-dihydroxynaphthalin.

Ganz besonders bevorzugte Diphenole (b) sind Hydrochinon, Resorcin und 4,4'-Dihydroxydiphenyl.

Bevorzugte aromatische Hydroxycarbonsäure (c) sind z.B. Verbindungen der Formeln

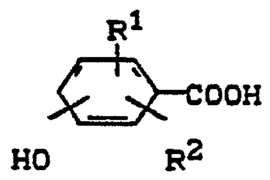
(V)

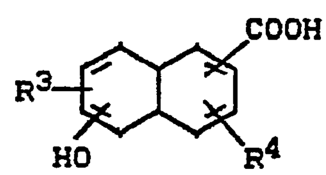
(VI)

worin
$R^1$ bis $R^4$ $C_1$-$C_4$-Alkyl, $C_1$-$C_4$-Alkoxy, $C_6$-$C_{10}$-Aryl oder -Aryloxy, $C_7$-$C_{12}$-Aralkyl (vorzugsweise Benzyl), Halogen (vorzugsweise Chlor und Brom) oder Wasserstoff bedeuten und die Bindungen zwischen Kern und Hydroxylgruppe bzw. Carboxylgruppe einen Winkel von 45° bis 180° bilden.

Besonders bevorzugte aromatische Hydroxycarbonsäuren (c) sind z.B. 4-Hydroxy-3-methylbenzoesäure, 4-Hydroxy-3-phenylbenzoesäure, 4-Hydroxy-2-ethylbenzoesäure, 3-Chlor-4-hydroxybenzoesäure, 3-Brom-4-hydroxybenzoesäure, 4-Hydroxy-3-methoxybenzoesäure, 4-Methyl-3-hydroxybenzoesäure, 4-Hydroxy-3-phenoxybenzoesäure, 6-Hydroxy-5-chlor-2-naphtoesäure, 6-Hydroxy-5-methyl-2-naphthoesäure, 6-Hydroxy-5-methoxy-2-naphthoesäure, 6-Hydroxy-4,7-dichlor-2-naphthoesäure.

Ganz besonders bevorzugte aromatische Hydroxycarbonsäuren (c) sind unsubstituierte Hydroxycarbonsäuren, wie p-Hydroxybenzoesäure und 6-Hydroxy-2-naphthoesäure.

Nicht sämtliche Kombinationen der aufgezählten Ausgangsmaterialien ergeben LC-Polyester. Der Fachmann wird seine Auswahl jeweils anhand der oben zitierten Literatur treffen oder aber aufgrund seiner Erfahrung geeignete Kombinationen auswählen.

Als Endgruppen können die LC-Polyester -COOH-, -H, -OH, $-OC_6H_5$, Acyloxy oder von Kettenabbrechern herrührende Reste enthalten. Bevorzugte Kettenabbrecher sind monofunktionelle aromatische Hydroxylverbindungen, wie 4-Hydroxydiphenyl, p-Nonylphenol, 4-(1,1,3,3-Tetramethylbutyl)-phenol, β-Naphthol und aromatische Monocarbonsäuren, wie Diphenylcarbonsäuren und Naphthalincarbonsäuren. Endgruppen können in Mengen von 0,5 bis 5 Mol-%, bezogen auf die Summe von aromatischen Dicarbonsäureresten (a) und Diphenolresten (b), vorhanden sein.

Die LC-Polyester können auch durch drei- oder höherfunktionelle - vorzugsweise aromatische - Monomere verzweigt sein. Die Mengen verzweigender Verbindungen betragen in der Regel 0,1 bis 1 Mol-%, bezogen auf die Summe von aromatischen Dicarbonsäureresten (a) und Diphenolresten (b). Beispiele für bevorzugte Verzweigungsmittel sind Phloroglucin, 1,3,5-Benzoltricarbonsäure und 3,5-Dihydroxybenzoesäure.

Die LC-Polyester besitzen in der Regel eine inhärente Viskosität von mindestens 0,5, vorzugsweise von mindestens 1,0 dl/g (gemessen an einer Lösung von 5 mg Polyester/ml p-Chlorphenol bei 45°). Sollten Polyester in p-Chlorphenol unlöslich sein, wird angenommen, daß sie die angegebene Mindestviskosität besitzen.

Bevorzugte LC-Polymere sind auch vollaromatische LC-Polyestercarbonate, d.h. voll-aromatische Polyester - z.B. wie oben beschrieben - deren aromatische Dicarbonsäurereste teilweise, vorzugsweise zu 60 bis 100 Mol-%, insbesondere zu 60 bis 90, Mol-%, durch Carbonylgruppen ersetzt sind.

Bevorzugte voll-aromatische LC-Polyestercarbonate B sind Polykondensate auf Basis von

(a) gegebenenfalls substituierter) p-Hydroxybenzoesäure,
(b) Diphenol,
(c) Kohlensäure, gegebenenfalls
(d) aromatischer Dicarbonsäure und gegebenenfalls
(e) Kettenabbrecher,

wobei ein Teil der Diphenolreste (b) als 4,4'-Dihydroxybiphenylreste (f) vorliegt und - abgesehen von den Endgruppen - für die molaren Verhältnisse der Reste folgendes gilt:

$$a + b = 1,$$

$$b = c + d,$$

$$f = 0{,}1 \text{ bis } 0{,}9, \text{ vorzugsweise } 0{,}11 \text{ bis } 0{,}7, \text{ insbesondere } 0{,}125 \text{ bis } 0{,}4 \text{ und}$$

$$\frac{c}{c + d} = 0{,}6 \text{ bis } 1, \text{ vorzugsweise } 0{,}6 \text{ bis } 0{,}9;$$

a = 0,4 bis 0,8, vorzugsweise 0,6 bis 0,75,
b-f = 0,02 bis 0,53, vorzugsweise 0,06 bis 0,36, insbesondere 0,1 bis 0,35
c = 0,12 bis 0,6, vorzugsweise 0,175 bis 0,4,
d = 0 bis 0,24, vorzugsweise 0 bis 0,12 und
f = 0,02 bis 0,53, vorzugsweise 0,0275 bis 0,28, insbesondere 0,3 bis 0,16.

Bevorzugte (a) p-Hydroxybenzoesäuren sind Verbindungen der Formeln V und VI.

Bevorzugte (b) Diphenole sind Verbindungen der Formel IV.

Bevorzugte (d) aromatische Dicarbonsäure sind Verbindungen entsprechend Formel III.

Die voll-aromatischen LC-Polyestercrbonate könnnen beispielsweise die bei den voll-aromatischen LC-Polyestern erwähnten Endgruppen enthalten.

Die voll-aromatischen LC-Polyestercarbonate können die Einheiten (a) bis (d) und (f) in statistischer Verteilung oder in Blöcken enthalten.

Die voll-aromatischen LC-Polyestercarbonate besitzen in der Regel eine inhärente Viskosität von mindestens 0,5, vorzugsweise von mindestens 1,0 dl/g (gemessen an einer Lösung von 5 mg Polyestercarbonat/ml p-Chlorphenol bei 50°C).

Die LC-Polymeren können zwecks Verbesserung ihrer Eigenschaften einer Festphasennachkondensation unterworfen werden. Üblicherweise führt man diese für 1 bis 25 Stunden bei 200 bis 300°C und bei vermindertem Druck durch.

Wenn auch die als Lösungsmittel zu verwendenden Verbindungen I natürlich auch diejenigen LC-Polymeren lösen, bei denen bislang keine Lösungsschwierigkeiten bestanden, so erscheint die Verwendung der Verbindungen I doch dort am wertvollsten, wo es mit ihrer Hilfe erstmalig gelingt, als unlösliche bekannte LC-Polymere in Lösung zu bringen.

Als "unlöslich" wird in diesem Zusammenhang ein LC-Polymer dann bezeichnet, wenn sich von einer Probe von 0,1 g LC-Polymer in 1 ml Pentafluorphenol bei 100°C innerhalb von 24 Stunden weniger als 90 Gew.-% lösen.

Die LC-Polymeren lösen sich in den Verbindungen I oft recht langsam. Man kann den Lösevorgang jedoch durch erhöhte Temperatur des Lösungsmittels und/oder durch Zerkleinerung der LC-Polymeren beschleunigen. In der Regel genügt Schütteln oder Rühren bei Raumtemperatur für einige Stunden oder bei höherer Konzentration bis zu einigen Wochen; um den Vorgang zu beschleunigen, kann man das Lösungsmittel aber auch auf bis zu 120°C oder höher erhitzen.

Die erhaltenen Lösungen können - je nach Molekulargewicht und Kettensteifigkeit der LC-Polymeren - Konzentrationen von 0,01 bis 30, vorzugsweise von 0,1 bis 10, Gew.-% besitzen.

Die Verbindungen I lösen nicht nur LC-Polymere, sondern auch eine Vielzahl anderer hochmolekularer Verbindungen, wie z.B. Polyester wie Polyethylenterephthalat und Polybutylenterephthalat, Polyamide, Polycarbonat, Styrol/Acrylnitril-Copolymerisate und Polystyrol.

Die Lösungen der LC-Polymeren in den Verbindungen I gestatten das Lösungsspinnen von LC-Polymeren, bei denen dies bislang aufgrund ihrer Unlöslichkeit noch nicht möglich war. Da - wie oben gesagt - die Verbindungen I nicht nur LC-Polymere, sondern auch viele andere Poly mere lösen, können mit Hilfe der als Lösungsmittel verwendeten Verbindungen I auch Mischfasern hergestellt werden.

Die Lösungen der LC-Polymeren gestatten - als hochtemperatur-beständige und lösungsmittelbeständige Überzugs- bzw. Imprägniermittel verwendet - das Beschichten und Imprägnieren verschiedenartigster Substrate, wie Metall, Holz und Textilfasern. Weiterhin gestatten sie die Herstellung von Gießfolien. Wenn man aus Lösungen von Polymermischungen in Verbindungen I Folien gießt, wobei mindestens ein Polymer in einem anderen Lösungsmittel löslich ist, während mindestens ein Polymer darin unlöslich

ist, werden hochtemperatur-beständige und lösungsmittelbeständige Membranen zugänglich.

Schließlich gestatten die Verbindungen I auch die analytische Untersuchung der bislang als unlöslich geltenden LC-Polymeren. So ermöglichen sie beispielsweise Viskosimetrie, statistische und dynamische Lichtstreuung, Gelpermeationschromatographie, Fällungs- und Lösungsfraktionierung, osmometrische Messungen und NMR-Spektroskopie.

Die in den nachfolgenden Beispielen genannten Prozentangaben beziehen sich auf das Gewicht.

Beispiele

1. Herstellung von Fluormethylphenolen

a) Herstellung von 4-Trifluormethoxy-3-trifluormethylphenol

50 g 4-Trifluormethoxy-3-trifluormethylanilin werden in 400 ml Essigsäure und 50 ml Schwefelsäure durch Zutropfen von 74 g 40%iger Nitrosylschwefelsäure bei 5°C diazotiert. Anschließend wird für eine Stunde bei 20°C nachgerührt. Diese Diazoniumsalzlösung wird sodann zu einer auf 100°C erhitzten Mischung auf 400 ml 50%iger Schwefelsäure, 20 g Kupfersulfat und 2 g Kupferoxid zugetropft. Nach Ende der Zugabe wird das Phenol mit Wasserdampf übergetrieben, in der Vorlage mit Dichlormethan extrahiert und destilliert.

Man erhält 38 g Produkt (Kp: 80–3°C/20 mbar, $n_D^{20}$ : 1.4218).

b) Analog erhält man aus 4-Difluorchlormethoxy-3-trifluormethylanilin das 4-Difluorchlormethoxy-3-trifluormethylphenol (Kp: 105–8°C/20 mbar, $n_D^{20}$ : 1.4455).

c) Herstellung von 2,4,5-Tris-trifluormethylphenol

In 75 ml DMSO werden nacheinander 50 g tert.-Butanol, 30 g 2,4,5-Tris-trifluormethyl-chlorbenzol und 12 g Natriumhydroxid gelöst und anschließend für 8 Stunden am Rückfluß erhitzt. Nach Abkühlen wird auf 250 ml Wasser gegossen, mit Salzsäure sauer gestellt und das Produkt mit Toluol extrahiert, die Toluol-Lösung mit Wasser gewaschen und getrocknet. Durch Destillation werden 18 g Produkt erhalten (Kp: 121-4°C/20 mbar).

2. Herstellung von 3,5-Bis-trifluormethylphenol erfolgt gemäß US-PS 2 547 679, Sp. 4, Z. 12-25.

3. Löslichkeit von LC-Polymeren

a) eingesetzte LC-Polymere:

I. Voll-aromatischer Polyester aus
31,2 Mol p-Hydroxybenzoesäure,
14,4 Mol Isophthalsäure,
2,4 Mol Terephthalsäure,
14,4 Mol Hydrochinon und
2,4 Mol 4,4'-Dihydroxydiphenyl
mit einer Schmelzviskosität (gemessen bei 330°C und einer Schergeschwindigkeit von $10^3$ $sec^{-1}$) von 280 Pa.s.

II. Voll-aromatisches Polyestercarbonat mit
68 Gew.-Teilen Resten von p-Hydroxybenzoesäure,
28 Gew.-Teilen Hydrochinonresten,
29 Gew.-Teilen Carbonatstrukturen,
4 Gew.-Teilen Resten von 4,4'-Dihydroxydiphenyl und
2,7 Gew.-Teilen Terephthalsäureresten
mit einer Schmelzviskosität (gemessen bei 330°C und einer Schergeschwindigkeit von $10^3$ $sec^{-1}$) von 100 Pa.s.

III. ®Vectra B 900 (Handelsprodukt der Fa. Celanese), ein voll-aromatisches Polyesteramid aus 2-Hydroxynaphthoesäure, p-Aminophenol und Terephthalsäure mit einem Schmelzpunkt von ca. 290°C.

IV. ®Xydar SRT 300 (Handelsprodukt der Fa. Dartco), ein voll-aromatischer Polyester aus p-Hydroxybenzoesäure, Terephthalsäure und 4,4'-Dihydroxydiphenyl mit einem Schmelzpunkt von ca. 420°C.

b) Löslichkeit

| Produkt | Lösungsbedingungen Konzentration [g/ml] | Temperatur* [°C] | Zeit [Std.] | Lösungsmittel Pentafluorphenol | 3,5-Bis-trifluormethylphenol [BTFMP] | Grenzviskosität [dl/g] in BTFMP bei 120° C |
|---|---|---|---|---|---|---|
| I | 0,01 | 50; 25 | 12 | - | + | 2,37 |
| II | 0,01 | 50; 25 | 24 | - | + | 3,60 |
| III | 0,01 | 50; 25 | 48 | - | + | 4,10 |
| IV | 0,01 | 50; 25 | 48 | - | + | 8,90 |

* in Pentafluorphenol; in 3,5-Bis-trifluormethylphenol

4. Anwendung

Eine Lösung von ®Vectra B 900 in 3,5-Bis-trifluormethylphenol (1 gew.-%ig) wurde auf eine Glasplatte gegossen. Das Lösungsmittel wurde bei 160°C innerhalb von ca. 1 Minute abgedampft und die Folie wurde mit Hilfe einer Pinzette abgezogen (Dicke ca. 1 µm). Die erhaltene Folie war blasenfrei und erwies sich - mit dem Auge betrachtet - als optisch homogen.

**Patentansprüche**

1. Verwendung von Verbindungen der Formel

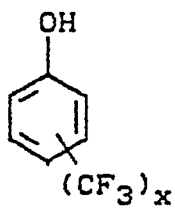

(I)

worin
x eine ganze Zahl von 1 bis 5 bedeutet, wobei die Verbindungen I auch zusätzlich bis zu (5–x) Substituenten aus der Reihe
(1) $C_1$–$C_4$-Alkyl,
(2) Halogen aus der Reihe Fluor, Chlor,
(3) Mono- oder Polyfluor-$C_1$–$C_4$-alkyl, das durch Wasserstoff-, Chlor- oder Bromatome substituiert sein kann,
(4) $C_1$–$C_4$-Alkoxy, das gegebenenfalls durch Fluor oder Chlor substituiert ist, tragen können,
als Lösungsmittel für LC-Polymere.

2. Verwendung nach Anspruch 1, dadurch gekennzeichnet, daß x 2 oder 3 bedeutet.

3. Verwendung nach Anspruch 1, dadurch gekennzeichnet, daß die Verbindung I
a) 1 bis 2 $CF_3$-Substituenten und
b)
i) 3 bis 4 Halogensubstituenten (am Kern),
ii) 1 bis 2 $C_1$–$C_4$-Alkoxygruppen,
iii) 1 bis 2 $CHF_2$- oder $CF_2Cl$-Gruppen oder
iv) 1 bis 2 $OCF_3$-Substituenten
tragen.

4. Verwendung nach Anspruch 1, dadurch gekennzeichnet, daß die Verbindung I 3,5-bis-trifluormethylphenol ist.

5. Trifluormethylphenole der Formel

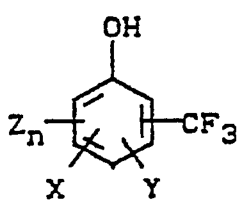

(II)

worin
X Fluor-$C_1$–$C_4$-alkyl oder Fluor-$C_1$–$C_4$-alkoxy,
Y Fluor, Chlor, Fluor-$C_1$–$C_4$-alkyl oder Fluor-$C_1$–$C_4$-alkoxy,
Z Fluor oder Chlor und
n Null, 1 oder 2 bedeuten, mit Ausnahme der Kombination X = $CF_3$, Y = Fluor oder Chlor und n = Null.

6. Trifluormethylphenole der Formel

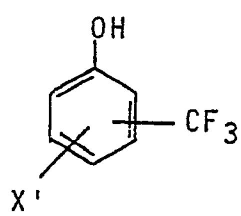

(IIa)

worin
X′ $OCF_2Cl$ bedeutet.

7. Trifluormethylphenol nach Anspruch 6 entsprechend der Formel

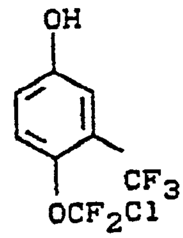

8. Trifluormethylphenol nach Anspruch 5 entsprechend der Formel

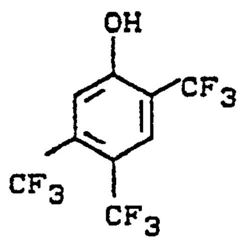

9. Verfahren zum Lösen von LC-Polymeren, dadurch gekennzeichnet, daß man als Lösungsmittel eine Verbindung der Formel

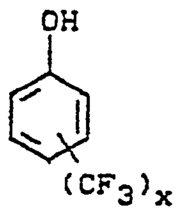

(I)

worin
x eine ganze Zahl von 1 bis 5 bedeutet, wobei die Verbindungen I auch zusätzlich bis zu (5–x) Substituenten aus der Reihe
(1) $C_1$–$C_4$-Alkyl,
(2) Halogen aus der Reihe Fluor, Chlor,
(3) Mono- oder Polyfluor-$C_1$–$C_4$-alkyl, das durch Wasserstoff-, Chlor- oder Bromatome substituiert sein kann,
(4) $C_1$–$C_4$-Alkoxy, das gegebenenfalls durch Fluor oder Chlor substituiert ist, tragen können,
einsetzt.

## Claims

1. Use of compounds of the formula

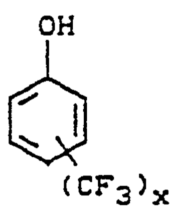

in which
x denotes an integer from 1 to 5, where the compounds I may also additionally carry up to (5–x) substituents from the series comprising
(1) $C_1$–$C_4$-alkyl
(2) halogen from the series comprising fluorine and chlorine,
(3) mono- or polyfluoro-$C_1$–$C_4$-alkyl which may be substituted by hydrogen, chlorine or bromine atoms,
(4) $C_1$–$C_4$-alkoxy which is optionally substituted by fluorine or chlorine,

as solvents for LC polymers.

2. Use according to Claim 1, characterized in that x denotes 2 or 3.

3. Use according to Claim 1, characterized in that the compounds I carry

a) 1 to 2 $CF_3$ substituents and

b)

i) 3 to 4 halogen substituents (in the nucleus),

ii) 1 to 2 $C_1$–$C_4$-alkoxy groups,

iii) 1 to 2 $-CHF_2$ or $-CF_2Cl$ groups or

iv) 1 to 2 $-OCF_3$ substituents.

4. Use according to Claim 1, characterized in that the compound I is 3,5-bis-trifluoromethylphenol.

5. Trifluoromethylphenols of the formula

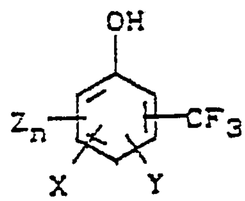

in which

X denotes fluoro-$C_1$–$C_4$-alkyl or fluoro-$C_1$–$C_4$-alkoxy,

Y denotes fluorine, chlorine, fluoro-$C_1$–$C_4$-alkyl or fluoro-$C_1$–$C_4$-alkoxy,

Z denotes fluorine or chlorine and n denotes zero, 1 or 2 except for the combination X = $CF_3$, Y = fluorine or chlorine and n = zero.

6. Trifluoromethylphenols of the formula

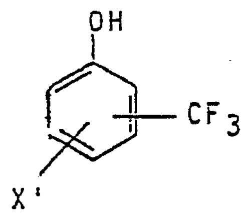

in which X′ denotes $OCF_2Cl$.

7. Trifluoromethylphenol according to Claim 6 corresponding to the formula

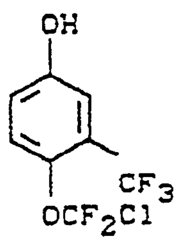

8. Trifluoromethylphenol according to Claim 5 corresponding to the formula

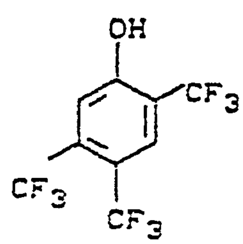

9. Process for dissolving LC polymers, characterized in that a compound of the formula

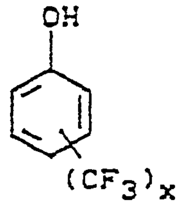

in which
x denotes an integer from 1 to 5, where the compounds I may also additionally carry up to (5–x) substituents from the series comprising
(1) $C_1$–$C_4$-alkyl,
(2) halogen from the series comprising fluorine and chlorine,
(3) mono- or polyfluoro-$C_1$–$C_4$-alkyl which may be substituted by hydrogen, chlorine or bromine atoms,
(4) $C_1$–$C_4$-alkoxy which is optionally substituted by fluorine or chlorine, is employed as a solvent.

**Revendications**

1. Utilisation de composés de formule

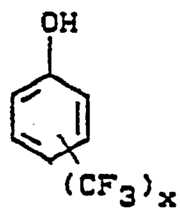

(I)

dans laquelle
x représente un entier de 1 à 5, les composés I pouvant également porter, en outre, jusqu'à (5–x) substituants choisis parmi le groupe comprenant
(1) un groupe alkyle en $C_1$–$C_4$,
(2) un atome d'halogène choisi parmi un atome de fluor et un atome de chlore,
(3) un groupe mono- ou polyfluoroalkyle en $C_1$–$C_4$, qui peut être substitué par un atome d'hydrogène, un atome de chlore ou un atome de brome,
(4) un groupe alcoxy en $C_1$–$C_4$ qui est éventuellement substitué par un atome de fluor ou un atome de chlore,
comme solvants pour des polymères LC.
2. Utilisation selon la revendication 1, caractérisée en ce que, x représente 2 ou 3.
3. Utilisation selon la revendication 1, caractérisée en ce que, les composés I portent
a) 1 à 2 substituants $CF_3$ et
b)
i) 3 à 4 substituants halogénés (sur le noyau)
ii) 1 à 2 groupes alcoxy en $C_1$–$C_4$,
iii) 1 à 2 groupes $CHF_2$ ou $CF_2Cl$ ou
iv) 1 à 2 substituants $OCF_3$.
4. Utilisation selon la revendication 1, caractérisée en ce que, le composé 1 est le 3,5-bis-trifluorométhylphénol.
5. Trifluorométhyl-phénols de formule:

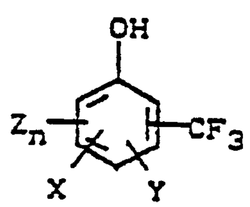

(II)

dans laquelle
X représente un groupe fluoroalkyle en $C_1$–$C_4$ ou un groupe fluoroalcoxy en $C_1$–$C_4$,
Y représente un atome de fluor, un atome de chlore, un groupe fluoroalkyle en $C_1$–$C_4$ ou un groupe fluoroalcoxy en $C_1$–$C_4$,
Z représente un atome de fluor ou un atome de chlore, et
n est égal à 0, 1 ou 2
en excluant la combinaison X = $CF_3$, Y = un atome de fluor ou un atome de chlore et n = zéro.

6. Trifluorométhylphénols de formule

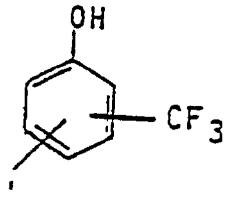

(IIa)

dans laquelle
X′ représente $OCF_2Cl$.

7. Trifluorométhylphénol selon la revendication 6, correspondant à la formule

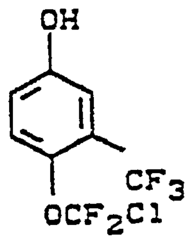

8. Trifluorométhylphénol selon la revendication 5, correspondant à la formule

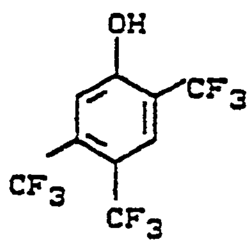

9. Procédé pour la dissolution de polymères LC, caractérisé en ce que, comme solvant, on met en œuvre un composé de formule

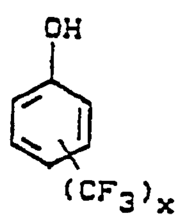

(I)

dans laquelle
x représente un entier de 1 à 5, les composés I pouvant également porter, en outre, jusqu'à (5–x) substituants choisis parmi le groupe comprenant

(1) un groupe alkyle en $C_1$–$C_4$,
(2) un atome d'halogène choisi parmi un atome de fluor et un atome de chlore,
(3) un groupe mono- ou polyfluoroalkyle en $C_1$–$C_4$, qui peut être substitué par un atome d'hydrogène, un atome de chlore ou un atome de brome,
(4) un groupe alcoxy en $C_1$–$C_4$ qui est éventuellement substitué par un atome de fluor ou un atome de chlore.